# EUROPÄISCHE PATENTSCHRIFT

(11) **EP 2 944 643 B1**
(45) Veröffentlichungstag und Bekanntmachung des Hinweises auf die Patenterteilung: **12.07.2017**
(21) Anmeldenummer: 15165635.2
(22) Anmeldetag: 29.04.2015
(51) Int. Cl.: C07F 7/18

(54) **HARNSTOFFHALTIGE MERCAPTOSILANE, VERFAHREN ZU DEREN HERSTELLUNG UND DEREN VERWENDUNG**
UREA-CONTAINING MERCAPTOSILANES, PROCESS FOR PREPARATION THEREOF AND USE THEREOF
MERCAPTOSILANES CONTENANT DE L'URÉE, LEUR PROCÉDÉ DE FABRICATION ET D'UTILISATION

(30) Priorität: 15.05.2014 DE 102014209233
(43) Veröffentlichungstag der Anmeldung: 18.11.2015
(73) Patentinhaber: Evonik Degussa GmbH, 45128 Essen (DE)
(72) Erfinder: Röben, Caren, 50670 Köln (DE); Moser, Ralph, 79104 Freiburg i. Br. (DE); Mayer, Stefanie, 79618 Rheinfelden (DE)

(56) Entgegenhaltungen:
- WO-A1-2013/087698
- JP-A- 2002 311 574

## Beschreibung

Die Erfindung betrifft harnstoffhaltige Mercaptosilane, Verfahren zur deren Herstellung sowie deren Verwendung.

Aus CAS 1082204-82-7, 1268617-33-9 und 104261-54-3 sind Verbindungen der Formel 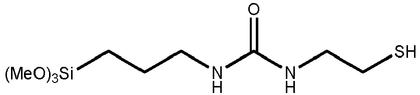 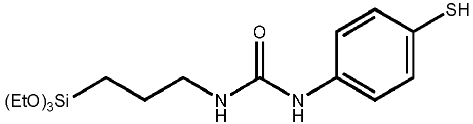 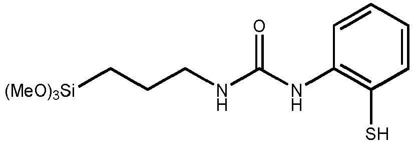 bekannt.

Ferner sind aus JP 2008279736 A harnstoffhaltige Silane für Beschichtungen bekannt. Aus DE 3424534 A1 sind N,N'- und N,N',N'-substituierte harnstoffhaltige Silane der Formel 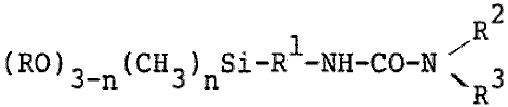 bekannt. Die Herstellung erfolgt durch Umsetzung einer Aminoverbindung, einem Halogensilan und Alkalicyanat in einem aprotisch, polaren organischem Lösungsmittel, beispielsweise DMF oder DMSO.

Ferner sind aus JP 2002311574 Pulverbeschichtungen bekannt enthaltend Silane der Formel R¹-S-R²-NH-C(O)-NH-R³-Si(R⁴)ₘ(OR⁵)₃₋ₘ.

Aus WO 9955754 A1 sind photosensitive Harzzusammensetzungen bekannt enthaltend Alkoxysilane der Formel

[(R¹O)₃₋ₐ(R²)ₐSi-R³-A-C(O)-B]ₘ-X.

Ferner sind aus WO 2013/087698 geblockte Mercaptosilane der Formel (HO)R¹₂Si-Z-S-C(=O)-A bekannt. Die geblockten Mercaptosilane enthalten Si-OH Gruppen.

Nachteile der bekannten harnstoffhaltigen Mercaptosilane sind das schlechte Verarbeitungsverhalten, die niedrige Netzwerkdichte, die schlechten Nassrutscheigenschaften und die niedrige dynamische Steifigkeit.

Aufgabe der vorliegenden Erfindung ist es harnstoffhaltige Mercaptosilane zur Verfügung zu stellen, welche gegenüber aus dem Stand der Technik bekannten harnstoffhaltigen Mercaptosilanen ein verbessertes Verarbeitungsverhalten, Netzwerkdichte, Nassrutscheigenschaften und dynamische Steifigkeit in Kautschukmischungen aufweisen. Gegenstand der Erfindung ist ein harnstoffhaltiges Mercaptosilan der Formel I 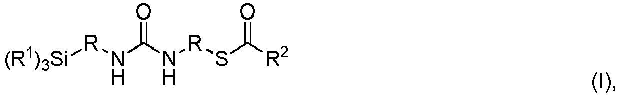 wobei R¹ gleich oder verschieden sind und C1-C10-Alkoxygruppen, vorzugsweise Methoxy- oder Ethoxygruppe, C2-C10- cyklische Dialkoxy-Gruppen, Phenoxygruppe, C4-C10-Cycloalkoxygruppen, C6-C20-Arylgruppen, vorzugsweise Phenyl, C1-C10-Alkylgruppen, vorzugsweise Methyl oder Ethyl, C2-C20-Alkenylgruppe, C7-C20-Aralkylgruppe oder Halogen, vorzugsweise Cl, R² eine einbindige C1-C20-Kohlenwasserstoffgruppe, vorzugsweise eine C1-C20-Alkylgruppe, C6-C20-Arylgruppe, C2-C20-Alkenylgruppe oder C7-C20-Aralkylgruppe, und R gleich oder verschieden sind und eine verzweigte oder unverzweigte, gesättigte oder ungesättigte, aliphatische, aromatische oder gemischt aliphatische/aromatische zweibindige C₁-C₃₀-, bevorzugt C₁-C₂₀-, besonders bevorzugt C₁-C₁₀-, ganz besonders bevorzugt C₁-C₇-, insbesondere bevorzugt C₂ und C₃-Kohlenwasserstoffgruppe, die gegebenenfalls mit F-, Cl-, Br-, I-, -CN oder HSsubstituiert ist, sind.

Harnstoffhaltige Mercaptosilane können Mischungen von harnstoffhaltigen Mercaptosilanen der Formel I sein.

Das Verfahrensprodukt kann Oligomere, die durch Hydrolyse und Kondensation der Alkoxysilanfunktionen der harnstoffhaltigen Mercaptosilanen der Formel I entstehen, enthalten. Die harnstoffhaltigen Mercaptosilane der Formel I können auf einem Träger, beispielsweise Wachs, Polymer oder Ruß, aufgebracht sein. Die harnstoffhaltigen Mercaptosilane der Formel I können auf einer Kieselsäure aufgebracht sein, wobei die Anbindung physikalisch oder chemisch erfolgt sein kann.

R kann bevorzugt -CH₂-, -CH₂CH₂-, -CH₂CH₂CH₂-, -CH₂CH₂CH₂CH₂-, -CH(CH₃)-, -CH₂CH(CH₃)-, - CH(CH₃)CH₂-, -C(CH₃)₂-, -CH(C₂H₅)-, -CH₂CH₂CH(CH₃)-, -CH(CH₃)CH₂CH₂-, -CH₂CH(CH₃)CH₂-, -CH₂CH₂CH₂CH₂CH₂-, -CH₂CH₂CH₂CH₂CH₂CH₂-, -CH₂CH₂CH₂CH₂CH₂CH₂CH₂-, -CH₂CH₂CH₂CH₂CH₂CH₂CH₂CH₂-, -CH₂CH₂CH₂CH₂CH₂CH₂CH₂CH₂CH₂-, -CH₂CH₂CH₂CH₂CH₂CH₂CH₂CH₂CH₂CH₂-, -CH₂CH₂CH₂CH₂CH₂CH₂CH₂CH₂CH₂CH₂CH₂CH₂-, -CH₂CH₂CH₂CH₂CH₂CH₂CH₂CH₂CH₂CH₂CH₂CH₂CH₂CH₂-, -CH₂CH₂CH₂CH₂CH₂CH₂CH₂CH₂CH₂CH₂CH₂CH₂CH₂CH₂CH₂CH₂-, -CH₂CH₂CH₂CH₂CH₂CH₂CH₂CH₂CH₂CH₂CH₂CH₂CH₂CH₂CH₂CH₂CH₂CH₂-oder 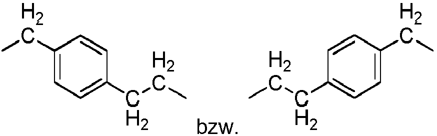 bedeuten.
R¹ kann bevorzugt Methoxy oder Ethoxy sein.

R² kann bevorzugt CH₃, CH₂CH₃, CH₂CH₂CH₃ oder (CH₂)₆CH₃ sein. Harnstofffhaltige Mercaptosilane der Formel I können bevorzugt sein:

(CH₃CH₂O)₃Si-CH₂-NH-CO-NH-CH₂-S-C(O)-CH₃,

(CH₃CH₂O)₃Si-CH₂CH₂-NH-CO-NH-CH₂-S-C(O)-CH₃,

(CH₃CH₂O)₃Si-CH₂-NH-CO-NH-CH₂CH₂-S-C(O)-CH₃,

(CH₃CH₂O)₃Si-CH₂CH₂-NH-CO-NH-CH₂CH₂-S-C(O)-CH₃,

(CH₃CH₂O)₃Si-CH₂CH₂CH₂-NH-CO-NH-CH₂-S-C(O)-CH₃,

(CH₃CH₂O)₃Si-CH₂CH₂CH₂-NH-CO-NH-CH₂CH₂-S-C(O)-CH₃,

(CH₃CH₂O)₃Si-CH₂-NH-CO-NH-CH₂CH₂CH₂-S-C(O)-CH₃,

(CH₃CH₂O)₃Si-CH₂CH₂-NH-CO-NH-CH₂CH₂CH₂-S-C(O)-CH₃,

(CH₃CH₂O)₃Si-CH₂CH₂CH₂-NH-CO-NH-CH₂CH₂CH₂-S-C(O)-CH₃,

(CH₃CH₂O)₃Si-CH₂-NH-CO-NH-CH₂-S-C(O)-(CH₂)₆CH₃,

(CH₃CH₂O)₃Si-CH₂CH₂-NH-CO-NH-CH₂-S-C(O)-(CH₂)₆CH₃,

(CH₃CH₂O)₃Si-CH₂-NH-CO-NH-CH₂CH₂-S-C(O)-(CH₂)₆CH₃,

(CH₃CH₂O)₃Si-CH₂CH₂-NH-CO-NH-CH₂CH₂-S-C(O)-(CH₂)₆CH₃,

(CH₃CH₂O)₃Si-CH₂CH₂CH₂-NH-CO-NH-CH₂-S-C(O)-(CH₂)₆CH₃,

(CH₃CH₂O)₃Si-CH₂CH₂CH₂-NH-CO-NH-CH₂CH₂-S-C(O)-(CH₂)₆CH₃,

(CH₃CH₂O)₃Si-CH₂-NH-CO-NH-CH₂CH₂CH₂-S-C(O)-(CH₂)₆CH₃,

(CH₃CH₂O)₃Si-CH₂CH₂-NH-CO-NH-CH₂CH₂CH₂-S-C(O)-(CH₂)₆OH₃,

(CH₃CH₂O)₃Si-CH₂CH₂CH₂-NH-CO-NH-CH₂CH₂CH₂-S-C(O)-(CH₂)₆CH₃,

(CH₃O)₃Si-CH₂-NH-CO-NH-CH₂-S-C(O)-CH₃,

(CH₃O)₃Si-CH₂CH₂-NH-CO-NH-CH₂-S-C(O)-CH₃,

(CH₃O)₃Si-CH₂-NH-CO-NH-CH₂CH₂-S-C(O)-CH₃,

(CH₃O)₃Si-CH₂CH₂-NH-CO-NH-CH₂CH₂-S-C(O)-CH₃,

(CH₃O)₃Si-CH₂CH₂CH₂-NH-CO-NH-CH₂-S-C(O)-CH₃,

(CH₃O)₃Si-CH₂CH₂CH₂-NH-CO-NH-CH₂CH₂-S-C(O)-CH₃,

(CH₃O)₃Si-CH₂-NH-CO-NH-CH₂CH₂CH₂-S-C(O)-CH₃,

(CH₃O)₃Si-CH₂CH₂-NH-CO-NH-CH₂CH₂CH₂-S-C(O)-CH₃,

(CH₃O)₃Si-CH₂CH₂CH₂-NH-CO-NH-CH₂CH₂CH₂-S-C(O)-CH₃,

(CH₃O)₃Si-CH₂-NH-CO-NH-CH₂-S-C(O)-(CH₂)₆CH₃,

(CH₃O)₃Si-CH₂CH₂-NH-CO-NH-CH₂-S-C(O)-(CH₂)₆CH₃,

(CH₃O)₃Si-CH₂-NH-CO-NH-CH₂CH₂-S-C(O)-(CH₂)₆CH₃,

(CH₃O)₃Si-CH₂CH₂-NH-CO-NH-CH₂CH₂-S-C(O)-(CH₂)₆CH₃,

(CH₃O)₃Si-CH₂CH₂CH₂-NH-CO-NH-CH₂-S-C(O)-(CH₂)₆CH₃,

(CH₃O)₃Si-CH₂CH₂CH₂-NH-CO-NH-CH₂CH₂-S-C(O)-(CH₂)₆CH₃,

(CH₃O)₃Si-CH₂-NH-CO-NH-CH₂CH₂CH₂-S-C(O)-(CH₂)₆CH₃,

(CH₃O)₃Si-CH₂CH₂-NH-CO-NH-CH₂CH₂CH₂-S-C(O)-(CH₂)₆CH₃,

(CH₃O)₃Si-CH₂CH₂CH₂-NH-CO-NH-CH₂CH₂CH₂-S-C(O)-(CH₂)₆OH₃.

Insbesondere bevorzugt ist die Verbindung der Formel

(EtO)₃Si-CH₂CH₂CH₂-NH-CO-NH-CH₂CH₂-S-C(O)-CH₃.

Ein weiterer Gegenstand der Erfindung ist ein Verfahren zur Herstellung der erfindungsgemäßen harnstoffhaltigen Mercaptosilane der Formel I 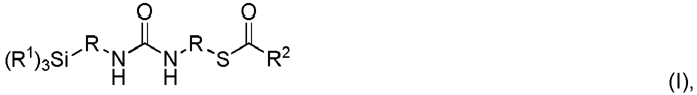 wobei R¹, R² und R die oben genannte Bedeutung haben, welches dadurch gekennzeichnet ist, dass man ein Halogensilan der Formel II 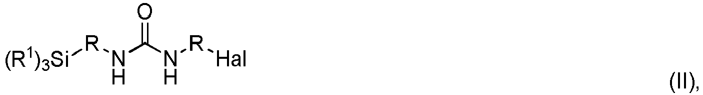 mit einer Verbindung der Formel III 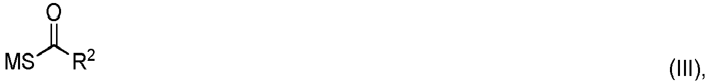 wobei R¹, R² und R die oben genannte Bedeutungen haben, Hal gleich F, Cl, Br oder I , vorzugsweise Cl, ist und M ein Alkalimetall, vorzugsweise K oder Na, ist,
in einem Alkohol umsetzt. Halogensilane der Formel II können bevorzugt sein:

(C₂H₅O)₃Si-CH₂-NH-C(O)-NH-CH₂-Cl,

(C₂H₅O)₃Si-CH₂CH₂-NH-C(O)-NH-CH₂-Cl,

(C₂H₅O)₃Si-CH₂CH₂CH₂-NH-C(O)-NH-CH₂-Cl,

(C₂H₅O)₃Si-CH₂-NH-C(O)-NH-CH₂CH₂-Cl,

(C₂H₅O)₃Si-CH₂CH₂-NH-C(O)-NH-CH₂CH₂-Cl,

(C₂H₅O)₃Si-CH₂CH₂CH₂-NH-C(O)-NH-CH₂CH₂-Cl,

(C₂H₅O)₃Si-CH₂-NH-C(O)-NH-CH₂CH₂CH₂-Cl,

(C₂H₅O)₃Si-CH₂CH₂-NH-C(O)-NH-CH₂CH₂CH₂-Cl,

(C₂H₅O)₃Si-CH₂CH₂CH₂-NH-C(O)-NH-CH₂CH₂CH₂-Cl,

(CH₃O)₃Si-CH₂-NH-C(O)-NH-CH₂-Cl,

(CH₃O)₃Si-CH₂CH₂-NH-C(O)-NH-CH₂-Cl,

(CH₃O)₃Si-CH₂CH₂CH₂-NH-C(O)-NH-CH₂-Cl,

(CH₃O)₃Si-CH₂-NH-C(O)-NH-CH₂CH₂-Cl,

(CH₃O)₃Si-CH₂CH₂-NH-C(O)-NH-CH₂CH₂-Cl,

(CH₃O)₃Si-CH₂CH₂CH₂-NH-C(O)-NH-CH₂CH₂-Cl,

(CH₃O)₃Si-CH₂-NH-C(O)-NH-CH₂CH₂CH₂-Cl,

(CH₃O)₃Si-CH₂CH₂-NH-C(O)-NH-CH₂CH₂CH₂-Cl oder

(CH₃O)₃Si-CH₂CH₂CH₂-NH-C(O)-NH-CH₂CH₂CH₂-Cl.

Verbindungen der Formel III können bevorzugt sein:

NaS-C(O)-CH₃,

NaS-C(O)-CH₂CH₃,

NaS-C(O)-CH₂CH₂CH₃,

NaS-C(O)-(CH₂)₆CH₃,

KS-C(O)-CH₃,

KS-C(O)-CH₂CH₃,

KS-C(O)-CH₂CH₂CH₃ oder

KS-C(O)-(CH₂)₆CH₃.

Bei dem erfindungsgemäßen Verfahren kann das Halogensilan der Formel II zur Verbindung der Formel II dosiert werden.

Bei dem erfindungsgemäßen Verfahren kann bevorzugt Verbindung der Formel III zu Halogensilan der Formel II dosiert werden.

Bei dem erfindungsgemäßen Verfahren kann das Halogensilan der Formel II zur Verbindung der Formel II im molekularen Verhältnis von 0,85:1 bis 1,15:1, bevorzugt 0,90:1 bis 1,10:1, besonders bevorzugt im Verhältnis 0,95:1 bis 1,05:1, eingesetzt werden.

Die Reaktion kann unter Luftausschluss durchgeführt werden.

Die Reaktion kann unter Schutzgasatmosphäre durchgeführt werden, zum Beispiel unter Argon oder Stickstoff, bevorzugt unter Stickstoff.

Das erfindungsgemäße Verfahren kann bei Normaldruck, erhöhtem Druck oder reduziertem Druck durchgeführt werden. Bevorzugt kann das erfindungsgemäße Verfahren bei Normaldruck durchgeführt werden.

Erhöhter Druck kann ein Druck von 1,1 bar bis 100 bar, bevorzugt von 1,5 bar bis 50 bar, besonders bevorzugt von 2 bar bis 20 bar und ganz besonders bevorzugt von 2 bis 10 bar, sein. Reduzierter Druck kann ein Druck von 1 mbar bis 1000 mbar, bevorzugt 1 mbar bis 500 mbar, besonders bevorzugt 1 mbar bis 250 mbar, ganz besonders bevorzugt 5 mbar bis 100 mbar, sein. Das erfindungsgemäße Verfahren kann zwischen 0°C und 150°C, bevorzugt zwischen 20°C und 100°C, besonders bevorzugt zwischen 50°C und 80°C, durchgeführt werden. Insbesondere bevorzugt kann das erfindungsgemäße Verfahren beim Siedepunkt des Alkohols durchgeführt werden.

Der bei dem erfindungsgemäßen Verfahren eingesetzte Alkohol kann vorzugsweise Methanol, Ethanol, Propanol, Butanol oder Cyclohexanol sein. Der Alkohol kann insbesondere bevorzugt Ethanol sein.

Der Alkohol kann nach der Reaktion entfernt, vorzugsweise abdestilliert, werden.

Das Reaktionsprodukt kann anschließend getrocknet werden. Die Trocknung kann bei Temperaturen von +20°C - +100°C, vorzugsweise von +25°C - +75°C, erfolgen. Die Trocknung kann bei Unterdruck von 1 - 500 mbar erfolgen.

Das durch das erfindungsgemäße Verfahren erhältliche harnstoffhaltige Mercaptosilan der Formel I 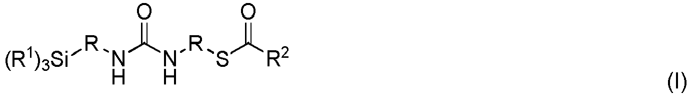 kann in einer Ausbeute von größer als 50%, bevorzugt größer als 60%, ganz besonders bevorzugt größer als 70%, erhalten werden.

In einer Ausführungsform kann das Halogensilan der Formel II vor der Umsetzung mit der Verbindung der Formel III aus dem Hydrochlorid-Salz eines Amins der Formel IV 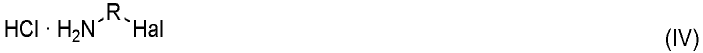 und Isocyanatsilan der Formel V 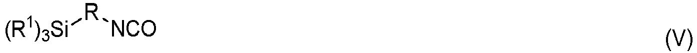 durch Zugabe einer Base, vorzugsweise NaOEt, hergestellt werden, wobei Hal, R¹ und R die oben genannten Bedeutungen haben.

Dabei kann die Base zugegeben werden bis sich ein pH-Wert zwischen 7 und 14 einstellt.

Bei dem erfindungsgemäßen Verfahren kann das Hydrochlorid-Salz der Amine der Formel IV zu Isocyantsilan der Formel V im molekularen Verhältnis von 0,85:1 bis 1,15:1, bevorzugt 0,90:1 bis 1,10:1, besonders bevorzugt im Verhältnis 0,95:1 bis 1,05:1, eingesetzt werden.

In der vorgenannten Ausführungsform kann das erfindungsgemäße Verfahren zur Herstellung von harnstoffhaltigen Mercaptosilanen der Formel I 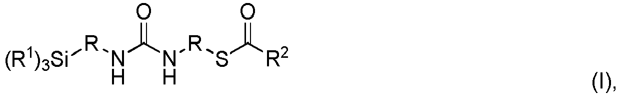 wobei R¹, R² und R die oben genannte Bedeutung haben, dadurch gekennzeichnet sein, dass man das Hydrochloridsalz des Amins der Formel IV 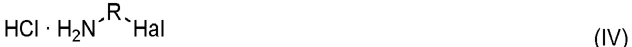 in Alkohol löst und mit einer Base umsetzt,
anschließend das Isocyanatsilan der Formel V 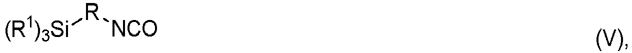 zugibt, und anschließend Verbindungen der Formel III 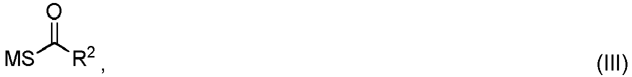 zugibt, filtriert und das Lösungsmittel entfernt, wobei Hal, M, R¹, R² und R die oben genannte Bedeutung haben.

In einer weiteren Ausführungsform kann das Halogensilan der Formel II vor der Umsetzung mit der Verbindung der Formel III aus der Isocyanathalogenverbindung der Formel VI 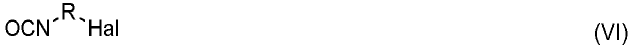 und Aminosilan der Formel VII 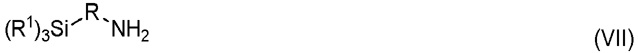 hergestellt werden, wobei Hal, R und R¹ die oben genannten Bedeutungen haben.

Die Umsetzung kann in einem Lösungsmittel, vorzugsweise Alkohol, besonders bevorzugt Ethanol, durchgeführt werden.

Bei dem erfindungsgemäßen Verfahren kann die Isocyanathalogenverbindung der Formel VI zu Aminosilan der Formel VII im molekularen Verhältnis von 0,85:1 bis 1,15:1, bevorzugt 0,90:1 bis 1,10:1, besonders bevorzugt im Verhältnis 0,95:1 bis 1,05:1, eingesetzt werden.

In der vorgenannten Ausführungsform kann das erfindungsgemäße Verfahren zur Herstellung von harnstoffhaltigen Mercaptosilanen der Formel I 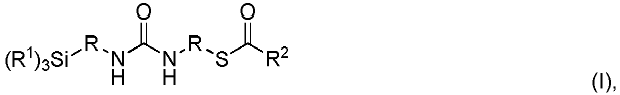 wobei R¹, R² und R die oben genannte Bedeutung haben, dadurch gekennzeichnet sein, dass man eine Isocyanathalogenverbindung der Formel VI 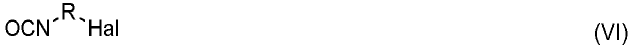 und Aminosilan der Formel VII 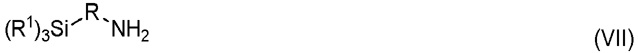 in Alkohol, vorzugsweise Ethanol, umsetzt, und anschließend eine Verbindung der Formel III 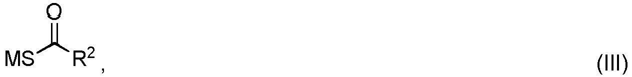 zugibt, filtriert und den Alkohol entfernt, wobei Hal, M, R, R¹ und R² die oben genannten Bedeutungen haben.

Das Produkt, hergestellt mit dem erfindungsgemäßen Verfahren, kann einen Restgehalt an Halogensilan der Formel II von weniger als 25 mol-%, bevorzugt weniger als 10 mol-%, besonders bevorzugt weniger als 5 mol-%, ganz besonders bevorzugt weniger als 3 mol-%, haben.

Die relativen mol-% des Halogensilans der Formel II in dem Produkt, hergestellt mit dem erfindungsgemäßen Verfahren, werden im ¹H-NMR durch Integration der H-Atome der -CH₂CH₂-Cl Gruppe des Halogensilans der Formel II gegen die H-Atome der Si-CH₂- Gruppe des harnstoffhaltigen Mercaptosilans der Formel I bestimmt.

Für die Substanz der Formel II (EtO)₃Si-CH₂CH₂CH₂-NH-CO-NH-CH₂CH₂-Cl wird zum Beispiel das Integral der H-Atome der -CH₂CH₂-Cl Gruppe (δ = 3.17 ppm) für die Bestimmung der relativen Gehalte verwendet.

Das Produkt, hergestellt mit dem erfindungsgemäßen Verfahren, kann einen Restgehalt an Hydrochlorid-Salz eines Amins der Formel IV von weniger als 25 mol-%, bevorzugt weniger als 10 mol-%, besonders bevorzugt weniger als 5 mol-%, ganz besonders bevorzugt weniger als 3 mol-%, haben.

Die relativen mol-% des Hydrochlorid-Salz eines Amins der Formel IV in dem Produkt, hergestellt mit dem erfindungsgemäßen Verfahren, werden im ¹³C-NMR durch Integration der C-Atome der - CH₂-NH_{2·}HCl Gruppe des Hydrochlorid-Salz eines Amins der Formel IV gegen die C-Atome der SiCH₂- Gruppe der harnstoffhaltigen Mercaptosilane der Formel I bestimmt.

Für die Substanz der Formel IV HCl·H₂N-CH₂-CH₂-Cl wird zum Beispiel das Integral der C-Atome der HCl·H₂N-CH₂-CH₂-Cl Gruppe (δ = 41,25 ppm) oder der HCl·H₂N-CH₂-CH₂-Cl Gruppe (δ = 40,79 ppm) für die Bestimmung der relativen Gehalte verwendet.

Das Produkt, hergestellt mit dem erfindungsgemäßen Verfahren, kann einen Restgehalt an Isocyanatsilan der Formel V von weniger als 10 mol-%, bevorzugt weniger als 5 mol-%, besonders bevorzugt weniger als 1 mol-%, ganz besonders bevorzugt weniger als 0,1 mol-%, haben.

Die relativen mol-% des Isocyanatsilan der Formel V in dem Produkt in einem Bereich >1 mol-%, hergestellt mit dem erfindungsgemäßen Verfahren, werden im ¹³C-NMR durch Integration der C-Atome der -NCO Gruppe des Isocyanatsilan der Formel V gegen die C-Atome der Si-CH₂- Gruppe der harnstoffhaltigen Mercaptosilane der Formel I bestimmt.

Für die Substanz der Formel V (EtO)₃Si-CH₂-CH₂-CH₂-NCO wird zum Beispiel das Integral der C-Atome der -NCO Gruppe (δ = 122,22 ppm) für die Bestimmung der relativen Gehalte in einem Bereich >1 mol-% verwendet.

Die relativen mol-% der Isocyanatsilan der Formel V in dem Produkt in einem Bereich <1 mol-%, hergestellt mit dem erfindungsgemäßen Verfahren, werden durch eine dem Fachmann bekannte quantitative FT-IR-Spektroskopie bestimmt. Die Kalibrierung der Methode erfolgt durch Verwendung von Kalibrierlösungen geeigneter Konzentration (z.B. in C₂Cl₄). Für die Messung werden ca. 1 g Probe in eine 25 ml Rollkragenflasche eingewogen und 25 g C₂Cl₄ zugegeben. Die Probe wird auf der Schüttelmaschine 1 - 2 Stunden geschüttelt. Im Anschluss wird die untere flüssige Phase vorsichtig in eine 20 mm IR-Küvette dosiert und per FT-IR-Spektroskopie vermessen (4000-1200 cm⁻¹, Auflösung 2 cm⁻¹). Unter gleichen Bedingungen wird ein Spektrum des Lösungsmittels zur Subtraktion aufgenommen.

Für die Substanz der Formel V (EtO)₃Si-CH₂-CH₂-CH₂-NCO wird zum Beispiel die Wellenlänge der Valenzschwingung der -NCO Gruppe bei 2270 cm⁻¹ für die Bestimmung der relativen Gehalte in einem Bereich <1 mol-% verwendet.

Das Produkt, hergestellt mit dem erfindungsgemäßen Verfahren, kann einen Restgehalt an Isocyanathalogenverbindung der Formel VI von weniger als 25 mol-%, bevorzugt weniger als 10 mol-%, besonders bevorzugt weniger als 5 mol-%, ganz besonders bevorzugt weniger als 3 mol-%, haben.

Die relativen mol-% der Isocyanathalogenverbindung der Formel VI in dem Produkt, hergestellt mit dem erfindungsgemäßen Verfahren, werden im ¹³C-NMR durch Integration der C-Atome der OCN-CH₂- Gruppe der Isocyanathalogenverbindung der Formel VI gegen die C-Atome der Si-CH₂-Gruppe des harnstoffhaltigen Mercaptosilans der Formel I bestimmt.

Für die Substanz der Formel VI OCN-CH₂-CH₂-Cl wird zum Beispiel das Integral der C-Atome der OCN-CH₂- Gruppe (δ = 124,33 ppm) für die Bestimmung der relativen Gehalte verwendet.

Das Produkt, hergestellt mit dem erfindungsgemäßen Verfahren, kann einen Restgehalt an Aminosilan der Formel VII von weniger als 10 mol-%, bevorzugt weniger als 5 mol-%, besonders bevorzugt weniger als 1 mol-%, ganz besonders bevorzugt weniger als 0,1 mol-%, haben.

Die relativen mol-% des Aminosilans der Formel VII in dem Produkt, hergestellt mit dem erfindungsgemäßen Verfahren, werden im ¹³C-NMR durch Integration der C -Atome der -CH₂-NH₂ Gruppe des Aminosilans der Formel VII gegen die C-Atome der Si-CH₂- Gruppe des harnstoffhaltigen Mercaptosilans der Formel I bestimmt.

Für die Substanz der Formel VII (EtO)₃Si-CH₂-CH₂-CH₂-NH₂ wird zum Beispiel das Integral der C-Atome der -CH₂-NH₂ Gruppe (δ = 45,15 ppm) für die Bestimmung der relativen Gehalte verwendet.

Harnstoffhaltiges Mercaptosilan der Formel I hergestellt nach dem erfindungsgemäßen Verfahren kann durch eine dem Fachmann bekannte ¹H-, ¹³C-, oder ²⁹Si-NMR Methode charakterisiert werden.

Der lösliche Anteil des harnstoffhaltigen Mercaptosilans der Formel I in dem durch die erfindungsgemäße Verfahren erhaltenen Produkte in DMSO-d⁶ oder CDCl₃ wird durch Zugabe eines internen Standards, wie beispielsweise Triphenylphosphinoxid (TPPO), in DMSO-d6 oder in CDCl₃ und eine dem Fachmann bekannte ¹H-NMR Methode ermittelt.

Die harnstoffhaltigen Mercaptosilane der Formel I können als Haftvermittler zwischen anorganischen Materialien, zum Beispiel
Glaskugeln, Glasplittern, Glasoberflächen, Glasfasern, oder oxidischen Füllstoffen, bevorzugt Kieselsäuren wie gefällten Kieselsäuren und pyrogene Kieselsäuren,
und organischen Polymeren, zum Beispiel Duroplasten, Thermoplasten oder Elastomeren, beziehungsweise als Vernetzungsmittel und Oberflächenmodifizierungsmittel für oxidische Oberflächen verwendet werden.

Die harnstoffhaltigen Mercaptosilane der Formel I können als Kopplungsreagenzien in gefüllten Kautschukmischungen, beispielsweise Reifenlaufflächen, technischen Gummiartikeln oder Schuhsolen, verwendet werden.

Vorteile der erfindungsgemäßen harnstoffhaltigen Mercaptosilane der Formel I sind verbessertes Verarbeitungsverhalten, Netzwerkdichte, Nassrutscheigenschaften und dynamische Steifigkeit in Kautschukmischungen.

### Beispiele

### Vergleichsbeispiel 1: Herstellung von (EtO)₃Si-CH₂CH₂CH₂-NH-CO-NH-CH₂CH₂-SH aus (EtO)₃SiCH₂CH₂CH₂-NH-CO-NH-CH₂CH₂-Cl und NaSH

Zu einer Lösung aus NaSH in Ethanol [hergestellt durch Einleiten von H₂S (15,21 g, 0,45 mol, 1,07 eq) in eine Natriumethanolat-Lösung (hergestellt aus Na (10,55 g, 0,46 mol, 1,10 eq) in EtOH (300 mL))] wird bei 52°C (EtO)₃Si-CH₂CH₂CH₂-NH-CO-NH-CH₂CH₂-Cl (138,90 g, 0,42 mol, 1,00 eq) in Ethanol (300 mL) zudosiert und auf 78°C erwärmt. Nach einer Reaktionszeit von 5 h wird auf Raumtemperatur gekühlt und die Suspension filtriert. Das Filtrat wird am Rotationsverdampfer vom Lösungsmittel befreit und am Vakuum getrocknet. Man erhält das Produkt (EtO)₃Si-CH₂CH₂CH₂-NH-CO-NH-CH₂CH₂-SH (134,96 g, 97,9% d.Th.) als weißen Feststoff.

¹H-NMR (δₚₚₘ, 500 MHz, CDCl₃): 0.64 (2H, t), 1.23 (9H, t), 1.36 (1H, br), 1.61 (2H, m), 2.67 (2H, t), 3.17 (2H, m), 3.37 (2H, m), 3.81 (6H, q), 4.74 (1H, br), 4.94 (1H, br);
¹³C-NMR (δₚₚₘ, 125 MHz, CDCl₃): 7.8 (1C), 18.3 (3C), 23.8 (1C), 25.6 (1C), 43.0 (1C), 43.5 (1C), 58.4 (3C), 158.9 (1C).

### Beispiel 1: Herstellung von (EtO)₃Si-CH₂CH₂CH₂-NH-CO-NH-CH₂CH₂-S-CO-CH₃ aus (EtO)₃Si-CH₂CH₂CH₂-NH-CO-NH-CH₂CH₂-Cl und KSAc

(EtO)₃Si-CH₂CH₂CH₂-NH-CO-NH-CH₂CH₂-Cl (81,52 g, 0,25 mol, 1,00 eq) wird in Ethanol (85 mL) in einem 500 mL Dreihalskolben mit Rührer, Rückflusskühler und Innenthermometer vorgelegt. Kaliumthioacetat (28,48 g, 0,25 mol, 1,00 eq) wird zugegeben und die Mischung zum Rückfluss erhitzt. Nach einer Reaktionszeit von 3,5 h wird auf Raumtemperatur gekühlt und die Suspension filtriert. Das Filtrat wird am Rotationsverdampfer vom Lösungsmittel befreit und am Vakuum getrocknet. Man erhält das Produkt (EtO)₃Si-CH₂CH₂CH₂-NH-CO-NH-CH₂CH₂-S-CH₃ (81,64 g, 89% d.Th.) als hellbraunen Feststoff.

¹H-NMR (δₚₚₘ, 500 MHz, CDCl₃): 0.64 (2H, t), 1.24 (9H, t), 1.61 (2H, m), 2.35 (3H, s), 3.01 (2H, t), 3.16 (2H, t), 3.34 (2H, t), 3.82 (6H, q), 4.5-7.0 (2H, br);
¹³C-NMR (δₚₚₘ, 125 MHz, CDCl₃): 7.8 (1C), 18.3 (3C), 23.8 (1C), 29.9 (1C), 30.6 (1C), 40.0 (1C), 43.0 (1C), 58.4 (3C), 159.0 (1C), 195.8 (1C).

### Beispiel 2: Herstellung von (EtO)₃Si-CH₂CH₂CH₂-NH-CO-NH-CH₂CH₂-S-CO-CH₃ aus (EtO)₃Si-CH₂CH₂CH₂-NH₂, OCN-CH₂CH₂-Cl und KSAc

3-Aminopropyltriethoxysilan (132,82 g, 0,60 mol, 1,00 eq) wird in Ethanol (300 mL) in einem Dreihalskolben mit KPG-Rührer, Innenthermometer, Tropftrichter und Rückflusskühler vorgelegt und auf-78°C gekühlt. 2-Chlorethylisocyanat (63,34 g, 0,60 mol, 1,00 eq) wird innerhalb von 1,75 h bei -78 bis -68°C zugetropft und danach auf 50°C erwärmt. Kaliumthioacetat (68,53 g, 0,60 mol, 1,00 eq) wird in fünf Portionen zugegeben und die Mischung zum Rückfluss erhitzt. Nach einer Reaktionszeit von 2,25 h wird auf Raumtemperatur gekühlt und die Suspension filtriert. Das Filtrat wird am Rotationsverdampfer vom Lösungsmittel befreit und am Vakuum getrocknet. Man erhält das Produkt (EtO)₃Si-CH₂CH₂CH₂-NH-CO-NH-CH₂CH₂-S-CH₃ (213,91 g, 97,3% d.Th.) als wachsartigen, weißen Feststoff.

¹H-NMR (δₚₚₘ, 500 MHz, CDCl₃): 0.64 (2H, t), 1.22 (9H, t), 1.62 (2H, m), 2.35 (3H, s), 3.01 (2H, t), 3.16 (2H, t), 3.34 (2H, t), 3.82 (6H, q), 4.7-5.0 (2H, br);
¹³C-NMR (δₚₚₘ, 125 MHz, CDCl₃): 7.8 (1C), 18.3 (3C), 23.8 (1C), 29.9 (1C), 30.6 (1C), 40.1 (1C), 43.0 (1C), 58.4 (3C), 158.7 (1C), 195.9 (1C).

### Beispiel 3: Herstellung von (EtO)₃Si-CH₂CH₂CH₂-NH-CO-NH-CH₂CH₂-S-CH₃ aus (EtO)₃Si-CH₂CH₂CH₂-NCO, HCl · H₂N-CH₂CH₂-Cl und KSAc

2-Chlorethylamin Hydrochlorid (73,86 g, 0,70 mol, 1,00 eq) wird in Ethanol (2,0 L) in einem 4 L Dreihalskolben mit KPG-Rührer, Innenthermometer, Tropftrichter und Rückflusskühler vorgelegt und auf -78°C gekühlt und Natriumethanolat (226,83 g, 0,70 mol, 1,00 eq, 21% in Ethanol) zugegeben. 3-Isocyanatopropyl(triethoxysilan) (173,15 g, 0,70 mol, 1,00 eq) wird nun innerhalb von 3 h bei -78 bis -65°C zugetropft und danach auf 50°C erwärmt. Kaliumthioacetat (79,95 g, 0,70 mol, 1,00 eq) wird in fünf Portionen zugegeben und die Mischung zum Rückfluss erhitzt. Nach einer Reaktionszeit von 4 h wird auf Raumtemperatur gekühlt und die Suspension filtriert. Das Filtrat wird am Rotationsverdampfer vom Lösungsmittel befreit und am Vakuum getrocknet. Man erhält das Produkt (EtO)₃Si-CH₂CH₂CH₂-NH-CO-NH-CH₂CH₂-S-CH₂CH₃ (288,02 g, quant.) als orangenes Öl.

### Beispiel 4: Kautschukmischungen

Die für die Kautschukmischungen verwendete Rezeptur ist in der folgenden Tabelle 1 angegeben. Dabei bedeutet die Einheit phr Gewichtsanteile bezogen auf 100 Teile des eingesetzten Rohkautschuks. Das erfinderische Silan wird isomolar zum Vergleichssilan eingesetzt.

**Tabelle 1**

| Substanz | Menge [phr] | Menge [phr] |
|---|---|---|
| 1. Stufe | Ref.kautschukmischung I enthaltend Vergl.beispiel 1 | Kautschukmischung II, enthaltend erf. Beispiel 2 |
| NR TSR^{a} | 10 | 10 |
| BR^{b} | 18 | 18 |
| SSBR^{c} | 72 | 72 |
| Kieselsäure^{d} | 95 | 95 |
| ZnO | 2,5 | 2,5 |
| Stearinsäure | 2,5 | 2,5 |
| TDAE Öl | 50 | 50 |
| Ozonschutzwachs | 2 | 2 |
| 6PPD^{e} | 2 | 2 |
| Vergl.beispiel 1 | 12 | - |
| Beispiel 2 | - | 14 |
| 2. Stufe | | |
| Batch | | |
| Stufe 2 | | |
| DPG^{†} | 2 | 2 |
| CBS^{g} | 2 | 2 |
| Schwefel | 2 | 2 |

| | | |
|---|---|---|
| Verwendete Substanzen: ^{a)} NR TSR: SIR 20 SED, Firma Aneka Bumi Pratama (TSR = Technically Specified Rubber; SIR = Standard Indonesian Rubber) ^{b)} BR: Polybutadien, Europrene Neocis BR 40, Firma Polimeri ^{c)} SSBR: Sprintan^{®} SLR-4601, Firma Styron ^{d)} Kieselsäure: ULTRASIL^{®} VN3 GR, Firma Evonik Industries AG ^{e)} 6PPD: N-(1,3-Dimethylbutyl)-N`-phenyl-p-phenylenediamine ^{f)} DPG: Diphenylguanidin ^{g)} CBS: N-Cyclohexyl-2-benzothiazolsufenamid | | |

Die Mischungsherstellung erfolgte unter üblichen Bedingungen in zwei Stufen in einem Laborkneter zur Herstellung von Gummimischungen (300 Milliliter bis 3 Liter Volumen), wobei zunächst in der ersten Mischstufe (Grundmischstufe) alle Bestandteile außer dem Vulkanisationssystem (Schwefel und vulkanisationsbeeinflussende Substanzen) für 200 bis 600 Sekunden bei 145 bis 165 °C, Zieltemperaturen von 152 bis 157 °C, vermischt werden. Durch Zugabe des Vulkanisationssystems in der zweiten Stufe (Fertigmischstufe) wird die Fertigmischung erzeugt, wobei für 180 bis 300 Sekunden bei 90 bis 120 °C gemischt wird.

Das allgemeine Verfahren zur Herstellung von Kautschukmischungen und deren Vulkanisate ist in "Rubber Technology Handbook", W. Hofmann, Hanser Verlag 1994 beschrieben.

Die gummitechnische Prüfung erfolgt gemäß den in Tabelle 2 angegebenen Prüfmethoden.

**Tabelle 2:**

| Physikalische Testung | Norm/ Bedingungen |
|---|---|
| Moving Die Rheometer (rotorloses Vulkameter): | ISO 6502 |
| *Minimales Drehmoment (dNm)* | ASTM D5289-12 |
| *Drehmomentsunterschied: Maximales Drehmoment-* | |
| *Minimales Drehmoment (dNm)* | |
| Rückprallelastizität bei 23°C *(%)* | DIN 53512 |
| Dynamisch-mechanische Messung bei 55°C *Dynamischer Speichermodul E' bei 0.15% Dehnung und bei 8% Dehnung (MPa)* | ISO 4664-1 |

Aus sämtlichen Mischungen wurden Prüfkörper durch Vulkanisation nach t₉₅ (gemessen am Moving Die Rheometer gemäß ISO 6502 / ASTM D5289-12) unter Druck bei 160°C hergestellt. In der Tabelle 3 sind die gummitechnischen Daten für die Vulkanisate angegeben.

**Tabelle 3:**

| Substanz | Ref.kautschukmischung I etnhaltend Vergl.beisp.1 | Kautschuk--mischung II, enthaltend erf. Beisp. 2 |
|---|---|---|
| Rohmischungsergebnisse: | | |
| Moving Die Methode: minimales Drehmoment nach 3. Stufe [dNm] | 4,4 | 3,5 |
| Moving Die Methode: Drehmomentsunterschied: Maximales Drehmoment- Minimales Drehmoment (dNm) nach 3. Stufe | 12 | 18 |
| Vulkanisatergebnisse: | | |
| Rückprallelast. 23°C [%] | 29 | 26 |
| Dynamisch-mechanische Messung bei 55°C E' bei 0,15% Dehnung (MPa) | 10,1 | 16,5 |
| Dynamisch-mechanische Messung bei 55°C E' bei 8% Dehnung (MPa) | 6,0 | 7,1 |
| Dynamisch-mechanische Messung bei 55°C E' bei 8% Dehnung - E' bei 0,15% Dehnung (MPa) | 4,1 | 9,4 |

Die Kautschukmischung II, die das erfindungsgemäße harnstoffhaltige Mercaptosilan aus Beispiel 2 enthält, zeigt ein verbessertes Verarbeitungsverhalten (niedrigeres minimales Drehmoment nach der 3. Mischstufe), eine erhöhte Netzwerkdichte (größere Differenz maximales Drehmomentminimales Drehmoment), verbesserte Nassrutscheigenschaften (Rückprallelastizität bei 23°C)sowie eine erhöhte dynamische Steifigkeit (E' bei 0.15% Dehnung, E' bei 8% Dehnung und E' bei 8% Dehnung - E' bei 0,15% Dehnung) gegenüber der Referenzkautschukmischung I, enthaltend das isomolar eingesetzte Vergleichsbeispiel 1 (harnstoffhaltiges Mercaptosilan).

## Patentansprüche

1. Harnstoffhaltiges Mercaptosilan der Formel I 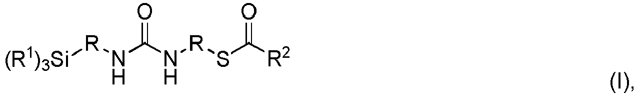 wobei R¹ gleich oder verschieden sind und C1-C10-Alkoxygruppen, C2-C10- cyklische Dialkoxy-Gruppen, Phenoxygruppe, C4-C10-Cycloalkoxygruppen, C6-C20-Arylgruppen, C1-C10-Alkylgruppen, C2-C20-Alkenylgruppe, C7-C20-Aralkylgruppe oder Halogen, R² eine einbindige C1-C20-Kohlenwasserstoffgruppe und R gleich oder verschieden sind und eine verzweigte oder unverzweigte, gesättigte oder ungesättigte, aliphatische, aromatische oder gemischt aliphatische/aromatische zweibindige C₁-C₃₀-Kohlenwasserstoffgruppe sind.

2. Harnstoffhaltiges Mercaptosilan gemäß Anspruch 1, **dadurch gekennzeichnet, dass** das harnstoffhaltige Mercaptosilan der Formel I
(CH₃CH₂O)₃Si-CH₂-NH-CO-NH-CH₂-S-C(O)-CH₃,
(CH₃CH₂O)₃Si-CH₂CH₂-NH-CO-NH-CH₂-S-C(O)-CH₃,
(CH₃CH₂O)₃Si-CH₂-NH-CO-NH-CH₂CH₂-S-C(O)-CH₃,
(CH₃CH₂O)₃Si-CH₂CH₂-NH-CO-NH-CH₂CH₂-S-C(O)-CH₃,
(CH₃CH₂O)₃Si-CH₂CH₂CH₂-NH-CO-NH-CH₂-S-C(O)-CH₃,
(CH₃CH₂O)₃Si-CH₂CH₂CH₂-NH-CO-NH-CH₂CH₂-S-C(O)-CH₃,
(CH₃CH₂O)₃Si-CH₂-NH-CO-NH-CH₂CH₂CH₂-S-C(O)-CH₃,
(CH₃CH₂O)₃Si-CH₂CH₂-NH-CO-NH-CH₂CH₂CH₂-S-C(O)-CH₃,
(CH₃CH₂O)₃Si-CH₂CH₂CH₂-NH-CO-NH-CH₂CH₂CH₂-S-C(O)-CH₃,
(CH₃CH₂O)₃Si-CH₂-NH-CO-NH-CH₂-S-C(O)-(CH₂)₆CH₃,
(CH₃CH₂O)₃Si-CH₂CH₂-NH-CO-NH-CH₂-S-C(O)-(CH₂)₆CH₃,
(CH₃CH₂O)₃Si-CH₂-NH-CO-NH-CH₂CH₂-S-C(O)-(CH₂)₆CH₃,
(CH₃CH₂O)₃Si-CH₂CH₂-NH-CO-NH-CH₂CH₂-S-C(O)-(CH₂)₆CH₃,
(CH₃CH₂O)₃Si-CH₂CH₂CH₂-NH-CO-NH-CH₂-S-C(O)-(CH₂)₆CH₃,
(CH₃CH₂O)₃Si-CH₂CH₂CH₂-NH-CO-NH-CH₂CH₂-S-C(O)-(CH₂)₆CH₃,
(CH₃CH₂O)₃Si-CH₂-NH-CO-NH-CH₂CH₂CH₂-S-C(O)-(CH₂)₆CH₃,
(CH₃CH₂O)₃Si-CH₂CH₂-NH-CO-NH-CH₂CH₂CH₂-S-C(O)-(CH₂)₆CH₃ oder
(CH₃CH₂O)₃Si-CH₂CH₂CH₂-NH-CO-NH-CH₂CH₂CH₂-S-C(O)-(CH₂)₆CH₃
ist.

3. Harnstoffhaltiges Mercaptosilan gemäß Anspruch 1, **dadurch gekennzeichnet, dass** das harnstoffhaltige Mercaptosilan der Formel I (EtO)₃Si-CH₂CH₂CH₂-NH-CO-NH-CH₂CH₂-S-C(O)-CH₃ ist.

4. Verfahren zur Herstellung von harnstoffhaltigem Mercaptosilan der Formel I gemäß Anspruch 1, **dadurch gekennzeichnet, dass** man ein Halogensilan der Formel II 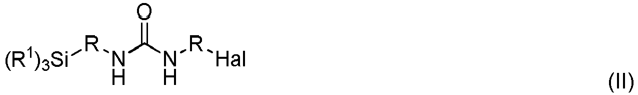 mit Verbindungen der Formel III 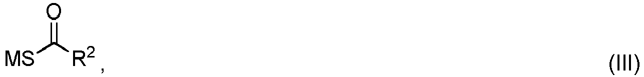 wobei R¹, R² und R die in Anspruch 1 genannten Bedeutungen haben, Hal gleich F, Cl, Br oder I ist und M ein Alkalimetall ist,
in einem Alkohol umsetzt.

5. Verfahren zur Herstellung von harnstoffhaltigem Mercaptosilan der Formel I gemäß Anspruch 4, **dadurch gekennzeichnet, dass** man die Reaktion unter Schutzgasatmosphäre durchführt.

6. Verfahren zur Herstellung von harnstoffhaltigem Mercaptosilan der Formel I gemäß Anspruch 4, **dadurch gekennzeichnet, dass** man die Reaktion bei Temperaturen zwischen 0°C und 150°C durchführt.

7. Verfahren zur Herstellung von harnstoffhaltigem Mercaptosilan der Formel I gemäß Anspruch 4, **dadurch gekennzeichnet, dass** man als Alkohol Ethanol einsetzt.

8. Verfahren zur Herstellung von harnstoffhaltigem Mercaptosilan der Formel I gemäß Anspruch 4, **dadurch gekennzeichnet ist, dass** man das Halogensilan der Formel II aus dem Hydrochlorid-Salz eines Amins der Formel IV 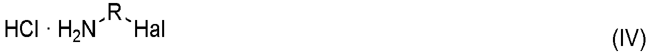 und Isocyanatsilan der Formel V 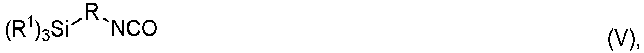 durch Zugabe einer Base herstellt, wobei Hal, R¹ und R die in Anspruch 4 genannten Bedeutungen haben.

9. Verfahren zur Herstellung von harnstoffhaltigem Mercaptosilan der Formel I gemäß Anspruch 8, **dadurch gekennzeichnet, dass** man als Base NaOEt einsetzt.

10. Verfahren zur Herstellung von harnstoffhaltigem Mercaptosilan der Formel I gemäß Anspruch 4, **dadurch gekennzeichnet ist, dass** man das Halogensilan der Formel II aus Isocyanathalogenverbindung der Formel VI 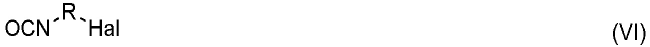 und Aminosilan der Formel VII 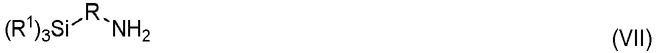 herstellt, wobei Hal, R und R¹ die in Anspruch 4 genannten Bedeutungen haben.

11. Verfahren zur Herstellung von harnstoffhaltigem Mercaptosilan der Formel I gemäß Anspruch 10, **dadurch gekennzeichnet, dass** man die Reaktion in Ethanol durchführt.

12. Verfahren zur Herstellung von harnstoffhaltigem Mercaptosilan der Formel I gemäß einem der Ansprüche 4 bis 10, **dadurch gekennzeichnet, dass** man den Alkohol abdestilliert.

13. Verfahren zur Herstellung von harnstoffhaltigem Mercaptosilan der Formel I gemäß Anspruch 12, **dadurch gekennzeichnet, dass** man das erhaltene Produkt trocknet.

## Claims

1. Urea-containing mercaptosilane of the formula I 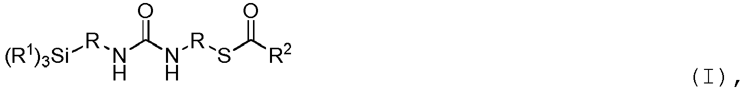 where R¹ are the same or different and are C1-C10 alkoxy groups, C2-C10 cyclic dialkoxy groups, phenoxy group, C4-C10 cycloalkoxy groups, C6-C20 aryl groups, C1-C10 alkyl groups, C2-C20 alkenyl group, C7-C20 aralkyl group or halogen, R² is a monovalent C1-C20 hydrocarbon group and R are the same or different and are a branched or unbranched, saturated or unsaturated, aliphatic, aromatic or mixed aliphatic/aromatic divalent C₁-C₃₀ hydrocarbon group.

2. Urea-containing mercaptosilane according to Claim 1, **characterized in that** the urea-containing mercaptosilane is of the formula I
(CH₃CH₂O)₃Si-CH₂-NH-CO-NH-CH₂-S-C(O)-CH₃,
(CH₃CH₂O)₃Si-CH₂CH₂-NH-CO-NH-CH₂-S-C(O)-CH₃,
(CH₃CH₂O)₃Si-CH₂-NH-CO-NH-CH₂CH₂-S-C(O)-CH₃,
(CH₃CH₂O)₃Si-CH₂CH₂-NH-CO-NH-CH₂CH₂-S-C(O)-CH₃,
(CH₃CH₂O)₃Si-CH₂CH₂CH₂-NH-CO-NH-CH₂-S-C(O)-CH₃,
(CH₃CH₂O)₃Si-CH₂CH₂CH₂-NH-CO-NH-CH₂CH₂-S-C(O)-CH₃,
(CH₃CH₂O)₃Si-CH₂-NH-CO-NH-CH₂CH₂CH₂-S-C(O)-CH₃,
(CH₃CH₂O)₃Si-CH₂CH₂-NH-CO-NH-CH₂CH₂CH₂-S-C(O)-CH₃,
(CH₃CH₂O)₃Si-CH₂CH₂CH₂-NH-CO-NH-CH₂CH₂CH₂-S-C(O)-CH₃,
(CH₃CH₂O)₃Si-CH₂-NH-CO-NH-CH₂-S-C(O)-(CH₂)₆CH₃,
(CH₃CH₂O)₃Si-CH₂CH₂-NH-CO-NH-CH₂-S-C(O)-(CH₂)₆CH₃,
(CH₃CH₂O)₃Si-CH₂-NH-CO-NH-CH₂CH₂-S-C(O)-(CH₂)₆CH₃,
(CH₃CH₂O)₃Si-CH₂CH₂-NH-CO-NH-CH₂CH₂-S-C(O)-(CH₂)₆CH₃,
(CH₃CH₂O)₃Si-CH₂CH₂CH₂-NH-CO-NH-CH₂-S-C(O)-(CH₂)₆CH₃,
(CH₃CH₂O)₃Si-CH₂CH₂CH₂-NH-CO-NH-CH₂CH₂-S-C(O)-(CH₂)₆CH₃,
(CH₃CH₂O)₃Si-CH₂-NH-CO-NH-CH₂CH₂CH₂-S-C(O)-(CH₂)₆CH₃, (CH₃CH₂O)₃Si-CH₂CH₂-NH-CO-NH-CH₂CH₂CH₂-S-C(O)-(CH₂)₆CH₃
or
(CH₃CH₂O)₃Si-CH₂CH₂CH₂-NH-CO-NH-CH₂CH₂CH₂-S-C(O)-(CH₂)₆CH₃.

3. Urea-containing mercaptosilane according to Claim 1, **characterized in that** the urea-containing mercaptosilane is of the formula I
(EtO)₃Si-CH₂CH₂CH₂-NH-CO-NH-CH₂CH₂-S-C(O)-CH₃.

4. Process for preparing urea-containing mercaptosilane of the formula I according to Claim 1, **characterized in that** a halosilane of the formula II 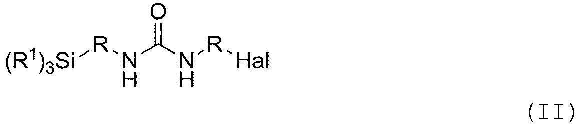 is reacted with compounds of the formula III 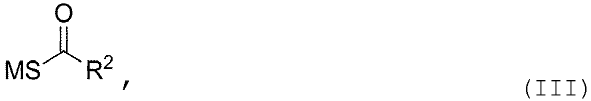 where R¹, R² and R are each as defined in Claim 1, Hal is F, Cl, Br or I and M is an alkali metal in an alcohol.

5. Process for preparing urea-containing mercaptosilane of the formula I according to Claim 4, **characterized in that** the reaction is conducted under a protective gas atmosphere.

6. Process for preparing urea-containing mercaptosilane of the formula I according to Claim 4, **characterized in that** the reaction is conducted at temperatures between 0°C and 150°C.

7. Process for preparing urea-containing mercaptosilane of the formula I according to Claim 4, **characterized in that** the alcohol used is ethanol.

8. Process for preparing urea-containing mercaptosilane of the formula I according to Claim 4, **characterized in that** the halosilane of the formula II is prepared from the hydrochloride salt of an amine of the formula IV 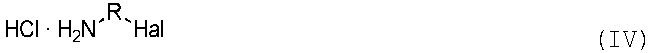 and isocyanatosilane of the formula V 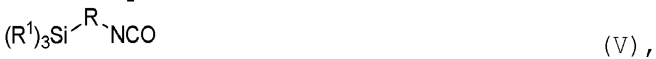 by addition of a base, where Hal, R¹ and R are each as defined in Claim 4.

9. Process for preparing urea-containing mercaptosilane of the formula I according to Claim 8, **characterized in that** the base used is NaOEt.

10. Process for preparing urea-containing mercaptosilane of the formula I according to Claim 4, **characterized in that** the halosilane of the formula II is prepared from isocyanate-halogen compound of the formula VI 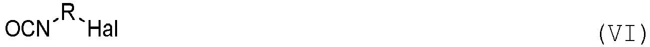 and aminosilane of the formula VII 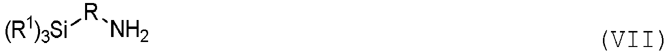 where Hal, R and R¹ are each as defined in Claim 4.

11. Process for preparing urea-containing mercaptosilane of the formula I according to Claim 10, **characterized in that** the reaction is conducted in ethanol.

12. Process for preparing urea-containing mercaptosilane of the formula I according to any of Claims 4 to 10, **characterized in that** the alcohol is distilled off.

13. Process for preparing urea-containing mercaptosilane of the formula I according to Claim 12, **characterized in that** the product obtained is dried.

## Revendications

1. Mercaptosilane, contenant de l'urée, de formule I 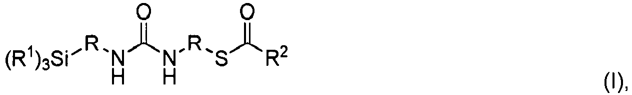 dans laquelle les radicaux R¹ sont identiques ou différents et représentent des groupes C₁-C₁₀-alcoxy, des groupes C₂-C₁₀-dialcoxy cycliques, le groupe phénoxy, des groupes C₄-C₁₀-cycloalcoxy, des groupes C₆-C₂₀-aryle, des groupes C₁-C₁₀-alkyle, des groupes C₂-C₂₀-alcényle, des groupes C₇-C₂₀-aralkyle ou halogène, R² représente un groupe hydrocarboné monovalent en C₁-C₂₀ et les radicaux R sont identiques ou différents et représentent un groupe hydrocarboné en C₁-C₃₀ divalent, ramifié ou non ramifié, saturé ou insaturé, aliphatique, aromatique ou aliphatique/aromatique mixte.

2. Mercaptosilane contenant de l'urée selon la revendication 1, **caractérisé en ce que** le mercaptosilane contenant de l'urée de formule I est
(CH₃CH₂O)₃Si-CH₂-NH-CO-NH-CH₂-S-C(O)-CH₃,
(CH₃CH₂O)₃Si-CH₂CH₂-NH-CO-NH-CH₂-S-C(O)-CH₃,
(CH₃CH₂O)₃Si-CH₂-NH-CO-NH-CH₂CH₂-S-C(O)-CH₃,
(CH₃CH₂O)₃Si-CH₂CH₂-NH-CO-NH-CH₂CH₂-S-C(O)-CH₃,
(CH₃CH₂O)₃Si-CH₂CH₂CH₂-NH-CO-NH-CH₂-S-C(O)-CH₃,
(CH₃CH₂O)₃Si-CH₂CH₂CH₂-NH-CO-NH-CH₂CH₂-S-C(O)-CH₃,
(CH₃CH₂O)₃Si-CH₂-NH-CO-NH-CH₂CH₂CH₂-S-C(O)-CH₃,
(CH₃CH₂O)₃Si-CH₂CH₂-NH-CO-NH-CH₂CH₂CH₂-S-C(O)-CH₃,
(CH₃CH₂O)₃Si-CH₂CH₂CH₂-NH-CO-NH-CH₂CH₂CH₂-S-C(O)-CH₃,
(CH₃CH₂O)₃Si-CH₂-NH-CO-NH-CH₂-S-C(O)-(CH₂)₆CH₃,
(CH₃CH₂O)₃Si-CH₂CH₂-NH-CO-NH-CH₂-S-C(O)-(CH₂)₆CH₃,
(CH₃CH₂O)₃Si-CH₂-NH-CO-NH-CH₂CH₂-S-C(O)-(CH₂)₆CH₃,
(CH₃CH₂O)₃Si-CH₂CH₂-NH-CO-NH-CH₂CH₂-S-C(O)-(CH₂)₆CH₃,
(CH₃CH₂O)₃Si-CH₂CH₂CH₂-NH-CO-NH-CH₂-S-C(O)-(CH₂)₆CH₃,
(CH₃CH₂O)₃Si-CH₂CH₂CH₂-NH-CO-NH-CH₂CH₂-S-C(O)-(CH₂)₆CH₃,
(CH₃CH₂O)₃Si-CH₂-NH-CO-NH-CH₂CH₂CH₂-S-C(O)-(CH₂)₆CH₃,
(CH₃CH₂O)₃Si-CH₂CH₂-NH-CO-NH-CH₂CH₂CH₂-S-C(O)-(CH₂)₆CH₃ ou
(CH₃CH₂O)₃Si-CH₂CH₂CH₂-NH-CO-NH-CH₂CH₂CH₂-S-C(O)-(CH₂) ₆CH₃.

3. Mercaptosilane contenant de l'urée selon la revendication 1, **caractérisé en ce que** le mercaptosilane contenant de l'urée de formule I est (EtO)₃Si-CH₂CH₂CH₂-NH-CO-NH-CH₂CH₂-S-C(O)-CH₃.

4. Procédé pour la préparation de mercaptosilane contenant de l'urée de formule I selon la revendication 1, **caractérisé en ce qu'**on transforme un halogénosilane de formule II 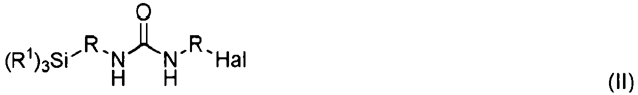 avec des composés de formule III 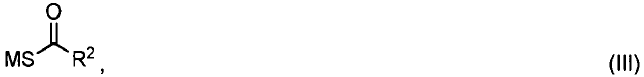 dans lesquelles R¹, R² et R présentent les significations mentionnées dans la revendication 1, Hal représente F, Cl, Br ou I et M est un métal alcalin, dans un alcool.

5. Procédé pour la préparation de mercaptosilane contenant de l'urée de formule I selon la revendication 4, **caractérisé en ce qu'**on réalise la réaction sous une atmosphère de gaz protecteur.

6. Procédé pour la préparation de mercaptosilane contenant de l'urée de formule I selon la revendication 4, **caractérisé en ce qu'**on réalise la réaction à des températures entre 0°C et 150°C.

7. Procédé pour la préparation de mercaptosilane contenant de l'urée de formule I selon la revendication 4, **caractérisé en ce qu'**on utilise, comme alcool, de l'éthanol.

8. Procédé pour la préparation de mercaptosilane contenant de l'urée de formule I selon la revendication 4, **caractérisé en ce qu'**on prépare l'halogénosilane de formule II à partir du sel de chlorhydrate d'une amine de formule IV 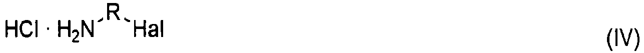 et d'isocyanatosilane de formule V 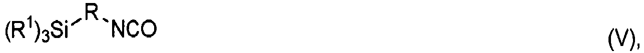 par addition d'une base, dans lesquelles Hal, R¹ et R présentent les significations mentionnées dans la revendication 4.

9. Procédé pour la préparation de mercaptosilane contenant de l'urée de formule I selon la revendication 8, **caractérisé en ce qu'**on utilise, comme base, du NaOEt.

10. Procédé pour la préparation de mercaptosilane contenant de l'urée de formule I selon la revendication 4, **caractérisé en ce qu'**on prépare l'halogénosilane de formule II à partir d'un composé d'isocyanatohalogène de formule VI 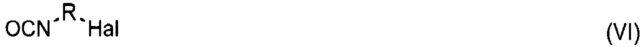 et d'un aminosilane de formule VII 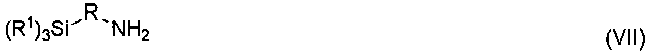 Hal, R et R¹ présentant les significations mentionnées dans la revendication 4.

11. Procédé pour la préparation de mercaptosilane contenant de l'urée de formule I selon la revendication 10, **caractérisé en ce qu'**on réalise la réaction dans de l'éthanol.

12. Procédé pour la préparation de mercaptosilane contenant de l'urée de formule I selon l'une quelconque des revendications 4 ou 10, **caractérisé en ce qu'**on élimine l'alcool par distillation.

13. Procédé pour la préparation de mercaptosilane contenant de l'urée de formule I selon la revendication 12, **caractérisé en ce qu'**on sèche le produit obtenu.
